# EUROPÄISCHE PATENTSCHRIFT

(11) **EP 2 265 571 B1**
(45) Veröffentlichungstag und Bekanntmachung des Hinweises auf die Patenterteilung: **04.03.2015**
(21) Anmeldenummer: 09723329.0
(22) Anmeldetag: 18.03.2009
(51) Int. Cl.: C07C 269/04, C07C 271/28

(54) **VERFAHREN ZUR HERSTELLUNG VON URETHANEN AUS MONO- UND DIFUNKTIONALEN AROMATISCHEN AMINEN**
METHOD FOR PRODUCING URETHANES COMPOSED OF MONO AND DI-FUNCTIONAL AROMATIC AMINES
PROCEDE DE FABRICATION D'URETHANES A PARTIR D'AMINES AROMATIQUES MONOFONCTIONNELLES ET DIFONCTIONNELLES

(30) Priorität: 18.03.2008 EP 08152939
(43) Veröffentlichungstag der Anmeldung: 29.12.2010
(73) Patentinhaber: BASF SE, 67056 Ludwigshafen (DE)
(72) Erfinder: LEITNER, Andreas, Pittsburg, PA 15212 (US); BAUMANN, Robert, 68529 Mannheim (DE); SIEGEL, Wolfgang, 67117 Limburgerhof (DE)
(86) Internationale Anmeldenummer: PCT/EP2009/053169
(87) Internationale Veröffentlichungsnummer: WO 2009/115538

(56) Entgegenhaltungen:
- EP-A- 0 048 371
- EP-A- 0 570 071
- DE-A1- 3 202 690
- TUNDO P ET AL: "Dimethyl Carbonate as an Ambident Electrophile" JOURNAL OF ORGANIC CHEMISTRY, AMERICAN CHEMICAL SOCIETY, EASTON.; US, Bd. 70, 1. Januar 2005 (2005-01-01), Seiten 2219-2224, XP002526970 ISSN: 0022-3263 [gefunden am 2005-02-17] in der Anmeldung erwähnt

## Beschreibung

Gegenstand der Erfindung ist ein Verfahren zur Herstellung von Urethanen durch Umsetzung von monofunktionalen und difunktionalen aromatischen Aminen mit einem Dialkylcarbonat in hohen Ausbeuten und Selektivitäten. Die so hergestellten Urethane können in weitere Folge zu technisch relevanten Isocyanaten umgesetzt werden.

Zur Herstellung von Urethanen ist eine Reihe von Verfahren bekannt.

In den Reaktionen werden beispielsweise Lewissäuren, wie z.B. Uransalze (US 3,763,217), Aluminiumspäne mit lod und Hg Promotoren (US 4,550,188), Zink-, Eisen-, Antimon- und Zinnsalze (US 4,268,683, 4,268,684, EP 391473) als Katalysatoren verwendet. Nachteilig für den industriellen Einsatz dieser Verfahren sind die zum Teil niedrigen Umsätze, niedrigen Selektivitäten oder beides.

Hohe Selektivitäten und Ausbeuten werden beispielsweise bei lewissäurekatalysierten Verfahren (Pb-Salze als Katalysatoren) erhalten, wenn ein hoher Überschuss an Dialkylcarbonat (Amin:Carbonat 1:20) eingesetzt wird (WO 98/55451, WO 98/56758). Der hohe Überschuss an Dialkylcarbonat führt zu großen Rückführströmen.

In anderen Fällen können hohe Ausbeuten an Urethan erreicht werden, wenn der in der Urethanisierung entstandene Harnstoff in einer zusätzlichen Reaktion thermisch in das entsprechende Urethan rückgespalten wird (EP 048371 (Katalysatoren: Blei-, Titan-, Zink- und Zirconsalze), EP 391473 (Katalysator: Zn-Salze). Die Rückspaltung erfordert einen zusätzlichen, energetisch aufwändigen Schritt.

Ein weiterer Nachteil bei der Anwendung von Lewissäuren als Homogenkatalysatoren sind die im Produkt verbleibenden Katalysatorrückstände, die nur unvollständig abgetrennt werden können.

In WO 2007/015852 wird der Einsatz von lewissauren Heterogenkatalysatoren für die Urethanisierung von aromatischen Aminen beschrieben. Dadurch entfällt eine aufwändige Abtrennung eines Homogenkatalysators. Die erhaltenen Umsätze sind für großtechnische Anwendungen zu niedrig und nehmen gemeinsam mit der Selektivität mit zunehmender Standzeit des heterogenen Katalysators ab.

Es ist weiterhin bekannt, Urethane aus aromatischen Aminen unter Verwendung von basischen Verbindungen, beispielsweise Alkalimetall- oder Erdalkalimetallalkoholaten herzustellen.

In DE 3202690 wird die Herstellung von aromatischen Urethanen durch Reaktion von Anilin und Dialkylcarbonaten in Gegenwart einer geringen Menge eines Metallalkoholates als Katalysator beschrieben. Die in den Beispielen beschriebenen Umsätze sind unvollständig und die erzielten Selektivitäten für eine industrielle Anwendung nicht ausreichend.

In Journal of Organic Chemistry, 2005, 70, 2219-2224 wird die Umsetzung von Anilin mit einem großen Überschuss an Dimethylcarbonat (40 facher Überschuss) in Gegenwart eines Überschusses an Base wie Natriummethylat (NaOMe) oder Kaliumtertiärbutylat (KO*t*Bu) beschrieben. Mit NaOMe wurde eine Selektivität von 67% nach einer Reaktionszeit von 210min erhalten. Mit KOtBu wird nach 1 min eine Selektivität von 100% beschrieben, die jedoch mit zunehmender Reaktionszeit auf 60% durch Bildung des Nebenproduktes *N*-Methylcarbanilat abfällt. Umsätze und isolierte Ausbeuten wurden nicht beschrieben.

Der Erfindung lag die Aufgabe zugrunde, ein Verfahren zur Herstellung von Urethanen aus monofunktionalen und difunktionalen aromatischen Aminen zu entwickeln, welches mit geringen molaren Überschüssen (bezogen auf die Aminogruppe) an Dialkylcarbonat eine Urethanisierungsreaktion in hohen Raum-Zeit-Ausbeuten und Selektivitäten ermöglicht. Die hergestellten Urethane sollen in weiterer Folge zu technisch wichtigen aromatischen Isocyanaten verarbeitet werden.

Überraschenderweise hat sich gezeigt, dass im Gegensatz zum Journal of Organic Chemistry, 2005, 70, 2219 (siehe Zeile 15, Seite 2) durch Umsetzung von aroamatischen Aminen mit Dialkylcarbonaten, deren Alkylrest 2-18, vorzugsweise 2-7 Kohlenstoffatome aufweisen, in Gegenwart von stöchiometrischen Mengen einer Base auch bei niedrigen Überschüssen an Dialkylcarbonat das gewünschte Urethan nach kurzer Reaktionszeit in exzellenten Ausbeuten (bis zu 98%) isoliert werden.

Gegenstand der Erfindung ist Verfahren zur Herstellung von Urethanen durch Umsetzung von aromatischen Aminen mit einem Dialkylcarbonat, dadurch gekennzeichnet, dass der Alkylrest des organischen Dialkylcarbonates 2-18, vorzugsweise 2-7 Kohlenstoffatome enthält, und die Umsetzung in Gegenwart einer Base durchgeführt wird, wobei die Base im molaren Verhältnis von 0,8 bis 1,2 bezogen auf die Aminogruppen, eingesetzt wird.

Vorzugsweise wird das Umsetzungsprodukt aus dem aromatischen Amin mit dem Dialkylcarbonat mit einer protischen Verbindung umgesetzt.

Unter einer protischen Verbindung wird eine Verbindung verstanden, die ein Proton übertragen kann.

Die protische Verbindung ist vorzugsweise ausgewählt aus der Gruppe, enthaltend Alkohole, Wasser und Mischungen aus beiden. Besonders bevorzugt ist die Verwendung von Wasser.

Das Dialkylcarbonat wird bevorzugt in einem molaren Verhältnis von Dialkylcarbonat zu Aminogruppen von 1:1 bis 10:1, besonders bevorzugt von 2:1 bis 3:1, eingesetzt.

Die Umsetzung des aromatischen Amins mit dem Dialkylcarbonat in Gegenwart der Base wird vorzugsweise bei einer Reaktionstemperatur von 60-150°C, besonders bevorzugt bei 100-140°C, durchgeführt. Bei dieser Temperatur kann innerhalb von 5-60 min ein quantitativer Umsatz des aromatischen Amins zu dem entsprechenden Urethan erhalten werden. Die Umsetzung wird üblicherweise bei Normaldruck durchgeführt.

Im erfindungsgemäßen Verfahren werden mono- und/oder difunktionelle aromatische Amine, die vorzugsweise keine Heteroatome im aromatischen Rest tragen, eingesetzt. Vertreter aus dieser Gruppe sind beispielsweise Anilin, o-, m-, p-Toluidin, o-, m-, p-Chloranilin und Isomerengemische, o-, m-, p-Bromanilin und Isomerengemische, o-, m-, p-Trifluormethylanilin und Isomerengemische, 2,4-, 2,6-, 3,4- und 3,5-Dimethyl-, -Dichlor-, -Dibrom- und -Diethylanilin und Isomerengemische, p-t-Butylanilin, Tolylendiamin (TDA), insbesondere 2,4- und 2,6-Tolylendiamin und deren Isomerengemische, Diaminophenylmethan (MDA), insbesondere 2,4'-Diaminophenylmethan, 4,4'-Diamino-phenylmethan, 2,2'-Diaminophenylmethan und höhere Homologe (Polyaminopolyphenylmethane)und deren Isomerengemische, und m-Phenylendiamin.

Bevorzugt werden die Isomeren von Toluylendiamin und/oder die Isomeren von Diaminophenylmethan eingesetzt.

Die Alkylkette des Dialkylcarbonats kann unverzweigt, verzweigt oder cyclisch sein. Vorzugsweise ist die Alkylkette verzweigt oder unverzweigt.

In einer Ausführungsform der Erfindung ist die Alkylkette des Dialkylcarbonats mit Heteroatomen modifiziert. Bei den Heteroatomen kann es sich um Halogenatome handeln, vorzugsweise um Fluoratome und/oder Chloratome, besonders bevorzugt um Fluoratome. In einer anderen Ausführungsform handelt es sich bei den Heteroatomen um Sauerstoffatome. Diese liegen bevorzugt als Ethergruppen vor.

In einer bevorzugten Ausführungsform der Erfindung sind die Dialkylcarbonate ausgewählt aus der Gruppe, enthaltend Diethylcarbonat, Di-n-Propylcarbonat, Di-n-butylcarbonat, Di-2-Methylpropylcarbonat, Di-3-Methylbutylcarbonat, Di-n-Pentylcarbonat, Bis-2-methoxyethylcarbonat, Bis-2-ethoxyethylcarbonat, Bis-2,2,2-Trifluorethylcarbonat, Diisobutylcarbonat, bevorzugt Diisobutylcarbonat und Di-n-butylcarbonat, besonders bevorzugt Düsobutylcarbonat.

Das Dialkylcarbonat kann vorzugsweise durch Umsetzung von Ethylencarbonat mit einem Alkohol hergestellt werden.

Bei der Base handelt es sich vorzugsweise um basische organische Metallverbindungen, insbesondere um Verbindungen von Alkalimetallen. Dabei kann es sich beispielsweise um Stickstoffatome enthaltende Verbindungen, beispielsweise Amide, wie Natriumamid oder Siliziumatome und Stickstoffatome enthaltende Verbindungen, beispielsweise Lithiumhexamethyldisilazid, handeln.

Besonders bevorzugt handelt es sich bei der Base um die Alkoholate von Alkalimetallen.

Der Alkohol des Metallalkoholats hat 2-18, besonders bevorzugt 2-7 Kohlenstoffatome in der Alkylkette. Die Alkylkette kann unverzweigt, verzweigt oder cyclisch sein.

In einer Ausführungsform der Erfindung ist die Alkylkette des entsprechenden Alkohols des Alkoholates mit Heteroatomen modifiziert. Bei den Heteroatomen kann es sich um Halogenatome handeln, vorzugsweise um Fluoratome und/oder Chloratome, besonders bevorzugt um Fluoratome. In einer anderen Ausführungsform handelt es sich bei den Heteroatomen um Sauerstoffatome. Diese liegen bevorzugt als Ethergruppen vor.

In einer besonders bevorzugten Ausführungsform des erfindungsgemäßen Verfahrens basieren die Dialkylcarbonate und die Metallalkoholate auf dem gleichen Alkohol. Dies hat den Vorteil, dass bei dem erfindungsgemäßen Verfahren eine geringere Menge an Verbindungen anwesend ist. Dadurch wird der Aufwand bei dem Verfahren verringert.

Die Umsetzung der aromatischen Amine mit dem Dialkylcarbonat in Gegenwart der Base wird vorzugsweise bei einer Reaktionstemperatur von 60-150°C, besonders bevorzugt bei 100-140°C, durchgeführt. Bei dieser Temperatur kann innerhalb von 5-60 min ein quantitativer Umsatz der aromatischen Amine erhalten werden. Die Umsetzung wird üblicherweise bei Normaldruck durchgeführt.

In einer bevorzugten Ausführungsform des erfindungsgemäßen Verfahrens zur Herstellung von Urethanen, bei der Wasser als protische Verbindung eingesetzt wird, umfasst das erfindungsgemäße Verfahren die Schritte
a) Umsetzung des aromatischen Amins mit dem Dialkylcarbonat in Gegenwart einer Base
b) Umsetzung der Reaktionsprodukte aus Schritt a mit Wasser
c) Trennung der in Schritt b) gebildeten Produkte und der wässrigen Base,
d) Umsetzung der wässrigen Base aus Schritt c) in die entsprechende nicht wässrige Base und deren Rückführung in Schritt a)
e) Isolierung des in Schritt c) abgetrennten Urethans.

Dieses Verfahren kann bevorzugt kontinuierlich durchgeführt werden.

Dabei wird in Schritt b) das Urethan gebildet.

Das Urethan kann dabei als Lösung in einem organischen Lösungsmittel oder als Reinstoff in Form einer Schmelze oder eines Feststoffes isoliert werden.

Bei den in Schritt b) gebildeten Produkten handelt es sich um das Urethan und, bei der Verwendung von Alkoholaten als Base, um das Alkoholat.

Der Verfahrensschritt a) wird in der Stufe 1 durchgeführt, der Verfahrensschritt b) in Stufe 2. bei diskontinuierlicher Fahrweise können die Stufen 1 und 2 im gleichen Reaktionsgefäß durchgeführt werden, bei kontinuierlicher Fahrweise vorzugsweise in unterschiedlichen Reaktionsgefäßen.

Das Produkt aus Stufe 1) kann ohne weitere Aufarbeitung in die Stufe 2) überführt werden.

In der Stufe 3 erfolgt die Umsetzung der in Stufe 2 anfallenden wässrigen in die nicht wässrige Base, im Fall der Verwendung von Metallalkoholaten die Umsetzung des Hydroxids in das Metallalkoholat. Dieses wird in die Stufe 1 zurückgeführt. Überschüssiger Alkohol, der in der Stufe 2 anfällt, wird dort ausgeschleust oder an einer anderen Stelle im Verfahren rückgeführt.

Das Produkt aus Stufe 2 wird, sofern es nicht bereits in dieser Form vorliegt, in eine nicht wässrige und eine wässrige Phase getrennt. Aus der organischen Phase, die das Urethan enthält, wird dieses abgetrennt und als Feststoff oder Schmelze isoliert oder direkt in dieser Form in weiteren Reaktionsstufen, beispielsweise in einer thermischen Spaltung zum entsprechenden Isocyanat, eingesetzt. Die abgetrennten Urethane können, sofern notwendig, gereinigt werden, beispielsweise durch Waschen.

In einer bevorzugten Ausführungsform des erfindungsgemäßen Verfahrens wird vor dem Schritt a) das Dialkylcarbonat durch Umsetzung von Ethylencarbonat mit einem Alkohol hergestellt.

In einer weiteren bevorzugten Ausführungsform des erfindungsgemäßen Verfahrens schließt sich an den Schritt e) als Schritt f) die Spaltung des Urethans zum Isocyanat und Alkohol an. Der in Schritt f) gebildete Alkohol kann dabei wieder in das Verfahren zurückgeführt werden. Die Rückführung kann beispielsweise in den Schritt d) oder in die vor Schritt a) stattfindende Herstellung der Dialkylcarbonate.

In dieser Erfindung konnte gezeigt werden, dass die erfindungsgemäße Umsetzung von aromatischen Aminen mit einem geringen Überschuss an Dialkylcarbonat, in hohen Selektivitäten und hohen Raum-Zeit- Ausbeuten möglich ist. Die Urethane werden in hohen Reinheiten gebildet, sodass keine aufwändige Nachreinigung erforderlich ist.

Die Erfindung soll in den nachstehenden Beispielen näher erläutert werden.

### Beispiel 1

In einen 21 Vierhalskolben, ausgestattet mit Rührer (Rührgeschwindigkeit 200min⁻¹), Innenthermometer und Argonüberleitung, wurden nacheinander 224,0g (1,3 mol) Diisobutylcarbonat, 39,2g (0,32 mol) 2,4-TDA und 64,8g (0,64 mol) Natriumisobutylat eingewogen und in ein auf 120°C vortemperiertes Ölbad getaucht. Die Analytik per Dünnschichtchromatographie nach 30 min zeigte quantitativen Umsatz des TDA. Der Kolbeninhalt wurde mit 1l Toluol verdünnt und 500ml Wasser bei 25°C zudosiert. Nach Phasentrennung wurde die organische Phase 3 mal mit 500ml Wasser gewaschen, die organische Phase anschließend über Na₂SO₄ getrocknet und das Toluol einrotiert.
Man erhielt leicht gelbliche Kristalle(100,1g) an reinem Urethan mit einer Ausbeute von 97%.

### Beispiel 2

In einen 250ml Vierhalskolben, ausgestattet mit Rührer, Innenthermometer und Argonüberleitung, wurden nacheinander 22,4g (0,13 mol) Diisobutylcarbonat, 3,9g (0,032 mol) 2,6-TDA und 6,5g (0,064 mol) Natriumisobutylat eingewogen und in ein auf 120°C temperiertes Ölbad getaucht. Die Analytik per Dünnschichtchromatographie nach 30 min zeigte quantitativen Umsatz des TDA. Der Kolbeninhalt wird mit 0,11 Toluol verdünnt und 50ml Wasser bei 25°C zudosiert. Nach Phasentrennung wurde die organische Phase 3 mal mit 50ml Wasser gewaschen, die organische Phase anschließend über Na₂SO₄ getrocknet und das Toluol einrotiert.
Man erhielt leicht gelbliche Kristalle(9,4g) an reinem Urethan mit einer Ausbeute von 92%,

### Beispiel 3

In einen 250ml Vierhalskolben, ausgestattet mit Rührer, Innenthermometer und Argonüberleitung, wurden nacheinander 224g (1,30 mol) Diisobutylcarbonat, 39,2g (0,32 mol) technisches TDA (Mischung aus 2,4/2,6-TDA = 80/20) und 64,8g (0,64 mol) Natriumisobutylat eingewogen und in ein auf 120°C temperiertes Ölbad getaucht. Die Analytik per Dünnschichtchromatographie nach 30 min zeigte quantitativen Umsatz des TDA. Der Kolbeninhalt wird mit 1l Toluoyl verdünnt und 500ml Wasser bei 25°C zudosiert. Nach Phasentrennung wurde die organische Phase 3 mal mit 500ml Wasser gewaschen, die organische Phase anschließend über Na₂SO₄ getrocknet und das Toluol einrotiert.
Man erhielt leicht gelbliche Kristalle(102 g) an reinem Urethan mit einer Ausbeute von 98%.

### Beispiel 4

In einen 2l Vierhalskolben, ausgestattet mit Rührer (Rührgeschwindigkeit 200min⁻¹), Innenthermometer und Argonüberleitung, wurden nacheinander 224,0g (1,3 mol) Di-n-butylcarbonat, 39,2g (0,32 mol) 2,4-TDA und 64,8g (0,64 mol) Natrium-n-butylat eingewogen und in ein auf 120°C vortemperiertes Ölbad getaucht. Die Analytik per Dünnschichtchromatographie nach 30 min zeigte quantitativen Umsatz des 2,4-TDA. Der Kolbeninhalt wurde mit 1l Toluol verdünnt und 500ml Wasser bei 25°C zudosiert. Nach Phasentrennung wurde die organische Phase 3 mal mit 500ml Wasser gewaschen, die organische Phase anschließend über Na₂SO₄ getrocknet und das Toluol einrotiert.
Man erhielt leicht gelbliche Kristalle(103,1g)an reinem Urethan mit einer Ausbeute von 99%.

### Beispiel 5

In einen 21 Vierhalskolben, ausgestattet mit Rührer (Rührgeschwindigkeit 200min⁻¹), Innenthermometer und Argonüberleitung, wurden nacheinander 284,1g (1,3 mol) Diisoamylcarbonat, 39,2g (0,32 mol) 2,4-TDA und 74,4g (0,65 mol) Natriumisoamylat eingewogen und in ein auf 120°C vortemperiertes Ölbad getaucht. Die Analytik per Dünnschichtchromatographie nach 30 min zeigte quantitativen Umsatz des TDA. Der Kolbeninhalt wurde mit 1l Toluol verdünnt und 100ml Wasser bei 25°C zudosiert. Nach Phasentrennung wurde die organische Phase 3 mal mit 100ml Wasser gewaschen, die organische Phase anschließend über Na₂SO₄ getrocknet und das Toluol einrotiert.
Man erhielt leicht gelbliche Kristalle(106,2g)an reinem Urethan mit einer Ausbeute von 94%.

### Beispiel 6

In einen 500ml Vierhalskolben, ausgestattet mit Rührer (Rührgeschwindigkeit 300min⁻¹), Innenthermometer und Argonüberleitung und Kühler wurden nacheinander 125,4 g (0,72 mol) Diisobutylcarbonat, 34,8g (0,18 mol) 4,4'-MDA und 36,4 g (0,38 mol) Natriumisobutylat eingewogen und in ein auf 120°C vortemperiertes Ölbad getaucht. Die Analytik per Dünnschichtchromatographie nach 30 min zeigte quantitativen Umsatz des 4,4'-MDA. Der Kolbeninhalt wurde mit 200 ml Toluol verdünnt und 34,2g Wasser zudosiert.. Nach 100 min Nachrührzeit wurde das Gemisch in einen 1000 ml Scheidetrichter überführt und die Phasen getrennt. Die organische Phase wurde eingeengt. Anschließend wurde der entstandene Feststoff bei 120°C und 0,25 mbar vollständig getrocknet. Man erhielt leicht gelbliche Kristalle (61 g) an reinem Urethan mit einer Ausbeute von 97%.

## Patentansprüche

1. Verfahren zur Herstellung von Urethanen durch Umsetzung von aromatischen Aminen mit einem Dialkylcarbonat, **dadurch gekennzeichnet, dass** der Alkylrest des organischen Dialkylcarbonates 2-18 Kohlenstoffatome enthält, und die Umsetzung in Gegenwart einer Base durchgeführt wird, wobei die Base im molaren Verhältnis von 0,8 bis 1,2, bezogen auf die Aminogruppen, eingesetzt wird.

2. Verfahren nach Anspruch 1, **dadurch gekennzeichnet, dass** die Base ein Metallalkoholat ist.

3. Verfahren nach Anspruch 1, **dadurch gekennzeichnet, dass** der Alkohol des Alkoholats 2-18 Kohlenstoffatome in der Kette aufweist.

4. Verfahren nach Anspruch 1, **dadurch gekennzeichnet, dass** der Alkohol Heteroatome in der Alkylkette aufweist.

5. Verfahren nach Anspruch 1, **dadurch gekennzeichnet, dass** die Alkylgruppen des Alkoholates linear oder verzweigt sind.

6. Verfahren nach Anspruch 1, **dadurch gekennzeichnet, dass** der Alkohol des Alkoholats der gleiche ist wie der des Dialkylcarbonats.

7. Verfahren nach Anspruch 1, **dadurch gekennzeichnet, dass** nach der Umsetzung der aromatischen Amine mit dem Dialkylcarbonat das erhaltene Reaktionsprodukt mit einer protischen Verbindung umgesetzt wird.

8. Verfahren nach Anspruch 3, **dadurch gekennzeichnet, dass** die protische Verbindung Wasser ist.

9. Verfahren nach Anspruch 1, **dadurch gekennzeichnet, dass** die aromatischen Amine eine Aminogruppe enthalten.

10. Verfahren nach Anspruch 1, **dadurch gekennzeichnet, dass** die aromatischen Amine zwei Aminogruppen enthalten.

11. Verfahren nach Anspruch 1, **dadurch gekennzeichnet, dass** die aromatischen Amine keine Heteroatome im aromatischen Ring aufweisen.

12. Verfahren nach Anspruch 1, **dadurch gekennzeichnet, dass** die aromatischen Amine ausgewählt sind aus der Gruppe, enthaltend Anilin, o-, m-, p-Toluidin, o-, m-, p-Chloranilin und Isomerengemische, o-, m-, p-Bromanilin und Isomerengemische, o-, m-, p-Trifluormethylanilin und Isomerengemische, 2,4-, 2,6-, 3,4- und 3,5-Dimethyl-, -Dichlor-, -Dibrom- und -Diethylanilin und Isomerengemische, p-t-Butylanilin, Tolylendiamin, Diaminophenylmethan, und höhere Homologe (Polyaminopolyphenylmethane) und m-Phenylendiamin.

13. Verfahren nach Anspruch 1, **dadurch gekennzeichnet, dass** die Dialkylcarbonate Heteroatome in der Alkylkette aufweisen.

14. Verfahren nach Anspruch 1, **dadurch gekennzeichnet, dass** die Dialkycarbonate ausgewählt sind aus der Gruppe, enthaltend Diethylcarbonat, Di-n-Propylcarbo-nat, Di-n-butylcarbonat, Di-2-Methylpropylcarbonat, Di-3-Methylbutylcarbonat, Di-n-Pentylcarbonat, Bis-2-methoxyethylcarbonat, Bis-2-ethoxyethylcarbonat, Bis-2,2,2-trifluorethylcarbonat.

15. Verfahren nach Anspruch 1, **dadurch gekennzeichnet, dass** das Dialkylcarbonat in einem molaren Verhältnis von Dialkylcarbonat zu Aminogruppen von 1:1 bis 10:1 eingesetzt wird.

16. Verfahren nach Anspruch 1, umfassend die Schritte
a) Umsetzung eines aromatischen Amins mit einem Dialkylcarbonat in Gegenwart einer Base
b) Umsetzung der Reaktionsprodukte aus Schritt a) mit Wasser
c) Trennung des in Schritt b) gebildeten Produkte und der wässrigen Base
d) Umsetzung der wässrigen Base aus Schritt c) in die entsprechende nicht wässrige Base und deren Rückführung in Schritt a)
e) Isolierung des in Schritt c) abgetrennten Urethans.

17. Verfahren nach Anspruch 16, **dadurch gekennzeichnet, dass** das in Schritt a) eingesetzte Dialkylcarbonat durch Umsetzung von Ethylencarbonat mit einem Alkohol hergestellt wird.

18. Verfahren nach Anspruch 16, **dadurch gekennzeichnet, dass** sich an Schritt e) die Spaltung f) des Urethans zum Isocyanat und Alkohol anschließt.

19. Verfahren nach Anspruch 16, **dadurch gekennzeichnet, dass** die in Schritt b) gebildete Lauge mit Alkohol zum entsprechenden Alkoholat umgesetzt und dieses wieder in den Schritt a) zurückgeführt wird.

20. Verfahren nach Anspruch 16, **dadurch gekennzeichnet, dass** das bei der Alkoholatbildung entstehende Wasser wieder in Schritt b) zurückgeführt wird.

## Claims

1. A process for preparing urethanes by reacting aromatic amines with a dialkyl carbonate, wherein the alkyl radical of the organic dialkyl carbonate comprises from 2 to 18 carbon atoms, and the reaction is carried out in the presence of a base, wherein the base is used in a molar ratio of from 0.8 to 1.2, based on the amino groups.

2. The process according to claim 1, wherein the base is a metal alkoxide.

3. The process according to claim 1, wherein the alcohol of the alkoxide has 2-18 carbon atoms in the chain.

4. The process according to claim 1, wherein the alcohol has heteroatoms in the alkyl chain.

5. The process according to claim 1, wherein the alkyl groups of the alkoxide are linear or branched.

6. The process according to claim 1, wherein the alcohol of the alkoxide is the same as that of the dialkyl carbonate.

7. The process according to claim 1, wherein, after the reaction of the aromatic amines with the dialkyl carbonate, the resulting reaction product is reacted with a protic compound.

8. The process according to claim 3, wherein the protic compound is water.

9. The process according to claim 1, wherein the aromatic amines comprise one amino group.

10. The process according to claim 1, wherein the aromatic amines comprise two amino groups.

11. The process according to claim 1, wherein the aromatic amines do not have any heteroatoms in the aromatic ring.

12. The process according to claim 1, wherein the aromatic amines are selected from the group comprising aniline, o-, m-, p-toluidine, o-, m-, p-chloroaniline and isomer mixtures, o-, m-, p-bromoaniline and isomer mixtures, o-, m-, p-trifluoromethylaniline and isomer mixtures, 2,4-, 2,6-, 3,4- and 3,5-dimethyl-, -dichloro-, -dibromo- and -diethylaniline and isomer mixtures, p-t-butylaniline, tolylenediamine, diaminophenylmethane, and higher homologs (polyaminopolyphenylmethanes) and m-phenylendiamine.

13. The process according to claim 1, wherein the dialkyl carbonates have heteroatoms in the alkyl chain.

14. The process according to claim 1, wherein the dialkyl carbonates are selected from the group comprising diethyl carbonate, di-n-propyl carbonate, di-n-butyl carbonate, di-2-methylpropyl carbonate, di-3-methylbutyl carbonate, di-n-pentyl carbonate, bis-2-methoxyethyl carbonate, bis-2-ethoxyethyl carbonate, bis-2,2,2-trifluoroethyl carbonate.

15. The process according to claim 1, wherein the dialkyl carbonate is used in a molar ratio of dialkyl carbonate to amino groups of from 1:1 to 10:1.

16. The process according to claim 1, comprising the steps of
a) reacting an aromatic amine with a dialkyl carbonate in the presence of a base
b) reacting the reaction products from step a) with water
c) separating the products formed in step b) and the aqueous base
d) converting the aqueous base from step c) to the corresponding nonaqueous base and recycling it to step a)
e) isolating the urethane removed in step c).

17. The process according to claim 16, wherein the dialkyl carbonate used in step a) is prepared by reacting ethylene carbonate with an alcohol.

18. The process according to claim 16, wherein step e) is followed by the cleavage f) of the urethane to the isocyanate and alcohol.

19. The process according to claim 16, wherein the alkali formed in step b) is reacted with the alcohol to give the corresponding alkoxide which is recycled back into step a).

20. The process according to claim 16, wherein the water formed in the alkoxide formation is recycled back into step b).

## Revendications

1. Procédé pour la préparation d'uréthanes par transformation d'amines aromatiques avec un carbonate de dialkyle, **caractérisé en ce que** le radical alkyle du carbonate de dialkyle organique contient 2-18 atomes de carbone et la transformation est réalisée en présence d'une base, la base étant utilisée dans un rapport molaire de 0,8 à 1,2 par rapport aux groupes amino.

2. Procédé selon la revendication 1, **caractérisé en ce que** la base est un alcoolate de métal.

3. Procédé selon la revendication 1, **caractérisé en ce que** l'alcool de l'alcoolate présente 2-18 atomes de carbone dans la chaîne.

4. Procédé selon la revendication 1, **caractérisé en ce que** l'alcool présente des hétéroatomes dans la chaîne alkyle.

5. Procédé selon la revendication 1, **caractérisé en ce que** les groupes alkyle de l'alcoolate sont linéaires ou ramifiés.

6. Procédé selon la revendication 1, **caractérisé en ce que** l'alcool de l'alcoolate est le même que celui du carbonate de dialkyle.

7. Procédé selon la revendication 1, **caractérisé en ce qu'**après la transformation des amines aromatiques avec le carbonate de dialkyle, le produit de réaction obtenu est transformé avec un composé protique.

8. Procédé selon la revendication 3, **caractérisé en ce que** le composé protique est de l'eau.

9. Procédé selon la revendication 1, **caractérisé en ce que** les amines aromatiques contiennent un groupe amino.

10. Procédé selon la revendication 1, **caractérisé en ce que** les amines aromatiques contiennent deux groupes amino.

11. Procédé selon la revendication 1, **caractérisé en ce que** les amines aromatiques ne contiennent pas d'hétéroatomes dans le cycle aromatique.

12. Procédé selon la revendication 1, **caractérisé en ce que** les amines aromatiques sont choisies dans le groupe contenant l'aniline, l'o-toluidine, la m-toluidine, la p-toluidine, l'o-chloroaniline, la m-chloroaniline, la p-chloroaniline et les mélanges d'isomères, l'o-bromoaniline, la m-bromoaniline, la p-bromoaniline et les mélanges d'isomères, l'otrifluorométhylaniline, la m-trifluorométhylaniline, la p-trifluorométhylaniline et les mélanges d'isomères, la 2,4-diméthylaniline, la 2,6-diméthylaniline, la 3,4-diméthylaniline et la 3,5-diméthylaniline, la 2,4-dichloroaniline, la 2,6-dichloroaniline, la 3,4-dichloroaniline et la 3,5-dichloroaniline, la 2,4-dibromoaniline, la 2,6-dibromoaniline, la 3,4-dibromoaniline et la 3,5-dibromoaniline, la 2,4-diéthylaniline, la 2,6-diéthylaniline, la 3,4-diéthylaniline et la 3,5-diéthylaniline et les mélanges d'isomères, la p-t-butylaniline, la toluylènediamine, le diaminophénylméthane et les homologues supérieurs (polyaminopolyphénylméthanes) et la m-phénylènediamine.

13. Procédé selon la revendication 1, **caractérisé en ce que** les carbonates de dialkyle présentent des hétéroatomes dans la chaîne alkyle.

14. Procédé selon la revendication 1, **caractérisé en ce que** les carbonates de dialkyle sont choisis dans le groupe contenant le carbonate de diéthyle, le carbonate de di-n-propyle, le carbonate de di-n-butyle, le carbonate de di-2-méthylpropyle, le carbonate de di-3-méthylbutyle, le carbonate de di-n-pentyle, le carbonate de bis-2-méthoxyéthyle, le carbonate de bis-2-éthoxyéthyle, le carbonate de bis-2,2,2-trifluoroéthyle.

15. Procédé selon la revendication 1, **caractérisé en ce que** le carbonate de dialkyle est utilisé dans un rapport molaire du carbonate de dialkyle aux groupes amino de 1:1 à 10:1.

16. Procédé selon la revendication 1, comprenant les étapes de
a) transformation d'une amine aromatique avec un carbonate de dialkyle en présence d'une base
b) transformation des produits de réaction de l'étape a) avec de l'eau
c) séparation du produit formé dans l'étape b) et de la base aqueuse
d) transformation de la base aqueuse de l'étape c) en la base non aqueuse correspondante et son recyclage dans l'étape a)
e) isolement de l'uréthane séparé dans l'étape c).

17. Procédé selon la revendication 16, **caractérisé en ce que** le carbonate de dialkyle utilisé dans l'étape a) est préparé par transformation de carbonate d'éthylène avec un alcool.

18. Procédé selon la revendication 16, **caractérisé en ce que** l'étape e) est suivie de la dissociation f) de l'uréthane en isocyanate et en alcool.

19. Procédé selon la revendication 16, **caractérisé en ce que** la lessive formée dans l'étape b) est transformée avec un alcool en alcoolate correspondant et celui-ci est à nouveau recyclé dans l'étape a).

20. Procédé selon la revendication 16, **caractérisé en ce que** l'eau formée lors de la formation de l'alcoolate est à nouveau recyclée dans l'étape b).
